# EUROPEAN PATENT APPLICATION

(11) **EP 4 119 084 A1**
(43) Date of publication of application: **18.01.2023**
(21) Application number: 22184306.3
(22) Date of filing: 12.07.2022
(51) Int. Cl.: A61B 18/14

(54) **ABLATION ELECTRODES MADE FROM ELECTRICAL TRACES OF FLEXIBLE PRINTED CIRCUIT BOARD**

(30) Priority: 13.07.2021 US 202117374849
(71) Applicant: Biosense Webster (Israel) Ltd, Yokneam, 2066717 (IL)
(72) Inventor: GOVARI, Assaf, Yokneam 2066717 (IL); BEECKLER, Christopher Thomas, Irvine 92618 (US); KEYES, Joseph Thomas, Irvine 92618 (US); HERRERA, kevin Justin, Irvine 92618 (US)
(74) Representative: Carpmaels & Ransford LLP

(57) **Abstract**

A catheter includes a shaft for insertion into an organ of a patient, an expandable distal-end assembly, and at least an electrical interconnection. The expandable distal-end assembly is coupled to the shaft and includes multiple splines, at least one of the splines includes a flexible substrate, which is configured to conform to tissue of the organ. The electrical interconnection has: (i) a first section, which is formed within the flexible substrate and is configured to conduct ablation signals, and (ii) a second section thicker than the first section, which is formed over an outer surface of the flexible substrate and is configured to apply the ablation signals to the tissue.

## Description

### FIELD OF THE INVENTION

The present invention relates generally to medical devices, and particularly to production methods and expandable catheters having ablation electrodes made from electrical traces.

### BACKGROUND OF THE INVENTION

Various techniques for producing expandable catheters having electrodes, have been published.

For example, U.S. Patent Application Publication 2009/0131930 describes a device positionable in a cavity of a bodily organ (e.g., a heart) that may discriminate between fluid (e.g., blood) and non-fluid tissue (e.g., wall of heart) to provide information or a mapping indicative of a position and/or orientation of the device in the cavity. Discrimination may be based on flow, or some other characteristic, for example electrical permittivity or force. The device may selectively ablate portions of the non-fluid tissue based on the information or mapping. The device may detect characteristics (e.g., electrical potentials) indicative of whether ablation was successful. The device may include a plurality of transducers, intravascularly guided in an unexpanded configuration and positioned proximate the non-fluid tissue in an expanded configuration. Expansion mechanism may include helical member(s) or inflatable member(s).

U.S. Patent Application Publication 2020/0061340 describes intravascular catheters that include a handle assembly with first and second handle actuators, where the actuators are adapted to separately control inner and outer catheter shafts in at least one of axial displacement, deflection, or rotation. The catheters may also include a medical tool secured to the outer shaft.

### SUMMARY OF THE INVENTION

An embodiment of the present invention that is described herein provides a catheter, including a shaft for insertion into an organ of a patient, an expandable distal-end assembly, and at least an electrical interconnection. The expandable distal-end assembly is coupled to the shaft and includes multiple splines, at least one of the splines includes a flexible substrate, which is configured to conform to tissue of the organ. The electrical interconnection has: (i) a first section, which is formed within the flexible substrate and is configured to conduct ablation signals, and (ii) a second section thicker than the first section, which is formed over an outer surface of the flexible substrate and is configured to apply the ablation signals to the tissue.

In some embodiments, the second section has a thickness larger than 0.1 mm. In other embodiments, the flexible substrate includes a flexible printed circuit board (FPCB), and the electrical interconnection includes an electrical trace made from gold.

In an embodiment, the FPCB includes at least an electrically insulating layer formed over the first section and configured to electrically insulate between the first section and the tissue. In another embodiment, the second section has at least a surface, which is not covered by the electrically insulating layer and is configured to apply the ablation signals to the tissue.

There is additionally provided, in accordance with an embodiment of the present invention, a method for producing a catheter, the method includes producing, in a flexible substrate, one or more electrical interconnections, at least one of the electrical interconnections is made from an electrically-conductive layer, including: (i) a first section formed within the flexible substrate, and (ii) a second section, thicker than the first section, which is formed over an outer surface of the flexible substrate. One or more stripes of the flexible substrate is cut for producing one or more splines of the catheter, and the one or more splines are assembled to a distal-end assembly of the catheter.

In some embodiments, assembling the one or more splines includes coupling (i) a proximal end of the spline to a proximal element of the distal-end assembly, and (ii) a distal end of the spline to a distal element of the distal-end assembly, the proximal element and the distal element are movable relative to one another for expanding and collapsing the distal-end assembly. In other embodiments, producing the electrical interconnection includes producing electrical traces made from a biocompatible layer. In yet other embodiments, the biocompatible layer includes gold.

In an embodiment, the electrically-conductive layer includes a first sub-layer and a second sub-layer, and producing the electrically-conductive layer includes: (i) forming the first sub-layer in the first and second sections, and (ii) forming the second sub-layer over the first sub-layer in the second section. In another embodiment, the first and second sub-layers are made from gold, and the first and second sub-layers have a combined thickness larger than 0.1 mm. In yet another embodiment, producing the electrically-conductive layer includes: (i) forming the electrically-conductive layer in the first and second sections, and (ii) thinning the electrically-conductive layer in the first section.

In some embodiments, the electrically-conductive layer includes gold and having a thickness larger than 0.1 mm. In other embodiments, the electrically-conductive layer includes gold and, after the thinning, the first section has a thickness smaller than 0.1 mm. In yet other embodiments, the method includes coupling the distal-end assembly to a shaft of the catheter.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a catheter-based position-tracking and ablation system, in accordance with an embodiment of the present invention;
Fig. 2 is schematic, pictorial illustrations of a distal-end assembly of the catheter in an expanded position, in accordance with embodiments of the present invention;
Fig. 2A is a cross-sectional view of section line A-A of Fig. 2 showing the first section and thicker second section;
Fig. 2B is an alternate cross-sectional view of the conductor 77 and electrode 88'; and
Fig. 3 is a flow chart that schematically illustrates a method for producing a basket catheter having ablation electrodes made from electrical traces, in accordance with an embodiment of the present invention.

### DETAILED DESCRIPTION OF EMBODIMENTS

### OVERVIEW

Embodiments of the present invention that are described hereinbelow provide improved techniques for producing an expandable catheter, such as a basket catheter, having ablation electrodes formed over splines made from a flexible substrate.

In some embodiments, an ablation catheter comprises a shaft for insertion into a patient heart, and an expandable distal end, in the present example, a basket-shaped distal-end assembly, also referred to herein as a basket. The basket is coupled to the shaft and is configured to be in a collapsed position when moved through the vasculature to or from the heart, and to be expanded to one or more expanded positions, e.g., for ablating tissue of the heart.

The basket comprises multiple arms, also referred to herein as splines. In some embodiments, at least one of (and typically all of) the splines comprises a flexible substrate, which is configured to conform to tissue of the heart when placed in contact therewith, so as to apply radiofrequency (RF) ablation signals (e.g., pulses) to the tissue via the aforementioned ablation electrodes.

In some embodiments, each spline of the basket comprises one or more electrical interconnections, typically made from gold or any other suitable type of electrically-conductive biocompatible material.

In some embodiments, each electrical interconnection comprises first and second sections. The first section is formed within the flexible substrate and is configured to conduct ablation signals, but is not exposed to the heart tissue.

In some embodiments, the second section of the electrical interconnection is thicker than the first section, e.g., having a thickness of about 0.5 mm or any other suitable thickness, and serves as an ablation electrode. The second section has an outer surface, which is exposed to the heart tissue and is configured to apply the ablation signals to the heart tissue. In principle, it is possible to couple an ablation electrode to the electrical interconnection, but having a thick trace at the second section simplifies and reduces the costs of producing process of the basket.

In some embodiments, the flexible substrate comprises a multi-layered flexible printed circuit board (FPCB) having an outer layer, which is configured to conform to the heart tissue. The FPCB may comprise multiple polymer layers. The outer layer is configured to electrically insulate between the first section and the tissue.

In some embodiments, the electrical interconnections are formed during the production of the FPCB by patterning gold traces. Each gold trace has the first section patterned on one side of a polymer layer of the FPCB substrate, whereas at least a surface of the second section, which is patterned on the opposite side of the polymer layer of the FPCB substrate, is exposed to and facing the tissue, so as to serve as an electrode. Using a common electrical trace for both conducting (by the first section) and applying the ablation signals (by the second section), may improve the flow of the ablation signals from an RF generator of the ablation system to the heart tissue.

The disclosed techniques improve the product quality and reduce the production costs of catheters having multiple electrodes, and in particular of expandable catheters, such as basket catheters, having a large amount (e.g., dozens, or hundreds, or thousands) of electrodes.

### SYSTEM DESCRIPTION

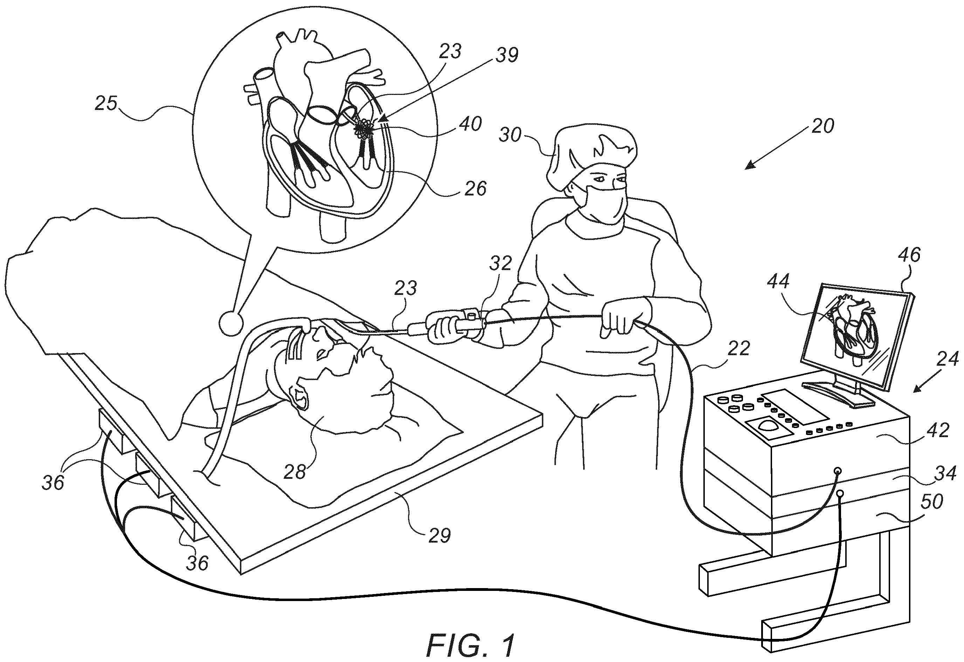

Fig. 1 is a schematic, pictorial illustration of a catheter-based position-tracking and ablation system 20, in accordance with an embodiment of the present invention. In some embodiments, system 20 comprises a catheter 22, in the present example an expandable cardiac catheter having a basket shape, and a control console 24. In the embodiment described herein, catheter 22 may be used for any suitable therapeutic and/or diagnostic purposes, such as but not limited to ablation of tissue in a heart 26.

In some embodiments, console 24 comprises a processor 42, typically a general-purpose computer, with suitable front end and interface circuits for receiving signals from catheter 22 and for controlling other components of system 20 described herein. Processor 42 may be programmed in software to carry out the functions that are used by the system, and is configured to store data for the software in a memory 50. The software may be downloaded to console 24 in electronic form, over a network, for example, or it may be provided on non-transitory tangible media, such as optical, magnetic or electronic memory media. Alternatively, some or all of the functions of processor 42 may be carried out using an application-specific integrated circuit (ASIC) or any suitable type of programmable digital hardware components.

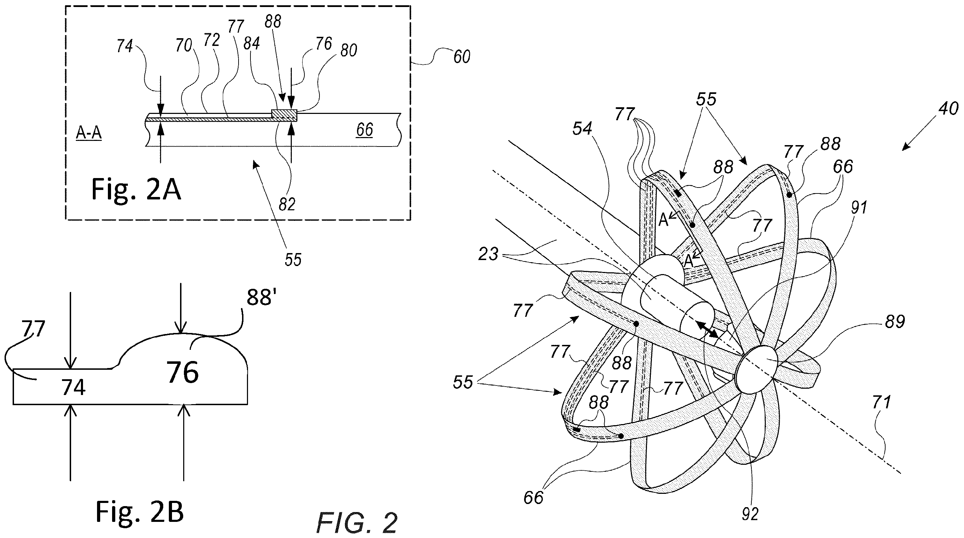

Reference is now made to an inset 25. In some embodiments, catheter 22 comprises a distal-end assembly 40 having multiple splines (shown in detail in Fig. 2 below), and a shaft 23 for inserting distal-end assembly 40 to a target location for ablating tissue in heart 26. During an ablation procedure, physician 30 inserts catheter 22 through the vasculature system of a patient 28 lying on a table 29. Physician 30 moves distal-end assembly 40 to the target location in heart 26 using a manipulator 32 near a proximal end of catheter 22, which is connected to interface circuitry of processor 42.

In some embodiments, catheter 22 comprises a position sensor 39 of a position tracking system, which is coupled to the distal end of catheter 22, e.g., in close proximity to distal-end assembly 40. In the present example, position sensor 39 comprises a magnetic position sensor, but in other embodiments, any other suitable type of position sensor (e.g., other than magnetic-based) may be used.

Reference is now made back to the general view of Fig. 1. In some embodiments, during the navigation of distal-end assembly 40 in heart 26, processor 42 receives signals from magnetic position sensor 39 in response to magnetic fields from external field generators 36, for example, for the purpose of measuring the position of distal-end assembly 40 in heart 26. In some embodiments, console 24 comprises a driver circuit 34, configured to drive magnetic field generators 36. Magnetic field generators 36 are placed at known positions external to patient 28, e.g., below table 29.

In some embodiments, processor 42 is configured to display, e.g., on a display 46 of console 24, the tracked position of distal-end assembly 40 overlaid on an image 44 of heart 26.

The method of position sensing using external magnetic fields is implemented in various medical applications, for example, in the CARTO^{™} system, produced by Biosense Webster Inc. (Irvine, Calif.) and is described in detail in U.S. Patents 5,391,199, 6,690,963, 6,484,118, 6,239,724, 6,618,612 and 6,332,089, in PCT Patent Publication WO 96/05768, and in U.S. Patent Application Publications 2002/0065455 A1, 2003/0120150 A1 and 2004/0068178 A1, whose disclosures are all incorporated herein by reference.

### DISTAL-END ASSEMBLY HAVING ABLATION ELECTRODES MADE FROM ELECTRICAL TRACES

Fig. 2 is schematic, pictorial illustrations of a distal-end assembly 40 of catheter 22 in an expanded position, in accordance with embodiments of the present invention.

In some embodiments, distal-end assembly 40 comprises an expandable basket catheter, which is coupled between shaft 23 and an apex 89 of distal-end assembly 40. Note that apex 89 is the distal-most part of catheter 22 which is typically (but not necessarily) orthogonal to an axis 71 of shaft 23.

In the present example, distal-end assembly 40 comprises multiple splines 55. Each spline 55 is made from a flexible substrate, such as a multi-layered flexible printed circuit board (FPCB) 66, or from any other suitable alloy or a multi-layered substrate. Note that FPCB 66 is selected for splines 55 for being (i) electrically conductive as will be described below, and (ii) sufficiently flexible to conform to the tissue of heart 26, which is intended to be ablated. In such embodiments, any other material selected for splines 55 has to be sufficiently flexible to conform to the aforementioned tissue when being placed in contact therewith.

In some embodiments, at least a given spline 55, and typically each spline 55 of distal-end assembly 40, has one or more electrical interconnections configured to conduct electrical signals and/or pulses. At least one of, and typically each electrical interconnection has (i) a first section, referred to herein as a conductor 77, and a second section, referred to herein as an electrode 88.

In some embodiments, each conductor 77 is formed within FPCB 66, e.g., between the polymer layers of FPCB 66, and is therefore shown in dashed lines. In the present example, conductor 77 is configured to conduct ablation signals from console 24 to electrode 88.

In some embodiments, electrode 88 is formed over the outer surface of FPCB 66 facing tissue of heart 26. When placed in contact with the tissue, electrode 88 is configured to apply the ablation signals (from console 24 via conductor 77) to the tissue. Electrode 88 may have any suitable shape, such as but not limited to a round shape, a rectangular shape, or a square shape.

In some embodiments, one spline 55 may comprise two or more electrical interconnections. In the present example, a first conductor 77 configured to conduct the ablation signals to a rectangular electrode 88, and a second longer conductor 77 configured to conduct the ablation signals to a round electrode 88, which is positioned closer to apex 89 relative to rectangular electrode 88. In other embodiments, all electrodes 88 of distal-end assembly 40 have the same shape. Moreover, each spline 55 may have any suitable number of electrodes 88 (e.g., between one and fifty) formed along the spline. In the example of Fig. 2, each ablation signal is routed to a separate electrode 88 using a separate conductor 77. In such embodiments, physician 30 controls both the position and type of signal applied to tissue.

In other embodiments, one conductor 77 may be connected to two or more electrodes 88 for conducting the same ablation signal to multiple electrodes 88. In such embodiments, physician 30 controls the type of ablation signal applied to the tissue, but the same signal is applied to multiple locations of the tissue at the same time.

In some embodiments, each spline 55 may have one or more electrodes positioned at the same distance from apex 89. In the present example, round electrodes 88 of each spline are positioned at the same first distance from apex 89. In such embodiments, physician 30 may apply to the tissue of an ostium (not shown) having a given diameter, a first set of ablation pulses. Similarly, all rectangular electrodes 88 are positioned at the same second (larger) distance from apex 89. In such embodiments, physician 30 may apply to the tissue of a different ostium (not shown) having a diameter smaller than that of the given diameter, a second set of ablation pulses. The first set and/or the second set may be applied to electrodes 88 at the same time or at different times.

Reference is now made to an inset 60 showing a sectional view A-A in Fig. 2A of one section of spline 55 marked with "A" arrows in the general view of Fig. 2.

In some embodiments, FPCB 66 comprises an outer layer 70 having an outer surface 72, which is configured to conform to the tissue intended to be ablated. In the present example, conductor 77 is made from gold or from any other suitable biocompatible electrically conductive layer, and has a thickness 74, e.g., from about 1 µm to about 50 µm, or any other suitable thickness.

In the context of the present disclosure and in the claims, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

In some embodiments, electrode 88 is made from the same material of conductor 77 and has a thickness 76 that is typically larger than thickness 74, e.g., from about 100 µm to about 1 mm, or any other suitable thickness. While the cross section of electrode 88 is shown as rectilinear in Fig. 2A, for conceptual understanding, it is noted that the cross-section of electrode 88 can be of any cross-section configurations such as, for example, elongated or curvilinear such that the electrode 88 can be an ovoid or a truncated oval electrode 88', such as shown in Fig. 2B. Where the cross-section of the electrode is non-constant, the thickness is measured at the maximum thickness of the cross-section of the electrode. As compared to the rectilinear (Fig. 2A) or square cross-section, the curvilinear or ovoid cross sections (electrode 88' in Fig. 2B) can be utilized to reduce stress concentrations on the electrodes 88' during the expansion or collapse of the spines 66. With these cross-sections in mind, it should be noted ratio of thickness 76 of the electrode 88 or 88' to the thickness 74 of conductive trace 77 can be selected from approximately 100:1 to approximately 20:1. That is, a second section (designated as 88) is approximately 20 to 100 times thicker than the first section (designated as conductor 77). As well, where the cross-section of the second section (i.e., electrode 88 or 88' being collectively designated as 88) devised as a curvilinear cross-section, the relevant thickness 76 for satisfying the ratio of the thickness of the second section to the first section (i.e., conductive trace thickness 74) is measured at the maximum thickness of the second section (or electrode 88).

In some embodiments, both conductor 77 and electrode 88 are made from a common (i.e., the same) trace, made from gold and having a different thickness at each section. As shown in inset 60, conductor 77 is buried within FPCB 66, and electrode 88 has two or more layers, in the present example, layers 80 and 82. Layer 82 is an extension of conductor 77, and layer 80 (disposed on layer 82) has a surface 84, which is exposed to the tissue intended to be ablated, so as to receive the ablation pulses from conductor 77 and to apply the ablation signal(s) to the aforementioned tissue. Note that layer 70 is physically and electrically insulating between conductor 77 and the tissue, so that the ablation pulses are applied to the tissue through surface 84.

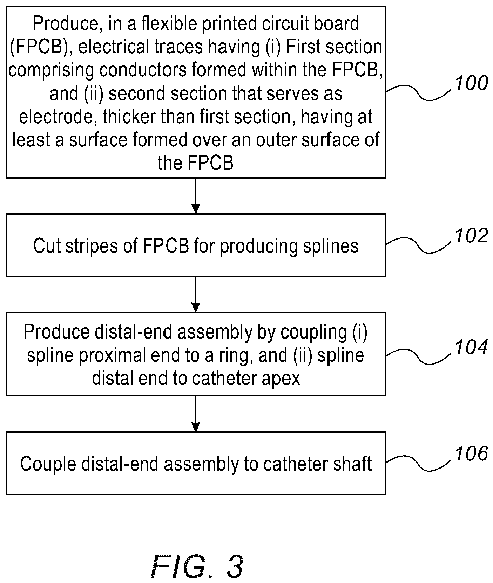

In some embodiments, conductor 77 and layer 82 are formed in one common process step, and layer 80 is formed over layer 82 using a different process step. In other embodiments, both conductor 77 and electrode 88 are made at the same time by forming an electrical trace having a thickness 76, and subsequently, thinning conductor 77 to thickness 74 and producing layer 70 over conductor 77. In both embodiments, conductor 77 and electrode 88 are made from the same electrical trace but have different thicknesses 74 and 76, respectively. The manufacturing process of distal-end assembly 40, and particularly of splines 55 is described in detail in Fig. 3 below.

Reference is now made back to the general view of Fig. 2. In some embodiments, when physician 30 moves distal-end assembly 40 of catheter 22 to the target location in heart 26, distal-end assembly 40 is in a collapsed position with all splines 55 straightened. When distal-end assembly 40 is positioned at the target location in heart 26, physician 30 typically reduces the distance between a ring 54 and apex 89 (e.g., by pulling apex 89 toward ring 54, or by pushing ring 54 and/or shaft 23 toward apex 89, or using any other technique), so as to have distal-end assembly 40 at an expanded position with splines 55 bent as shown in Fig. 2.

In some embodiments, distal-end assembly 40 comprises a bump stop 91, which is coupled to apex 89 and is configured to control a minimal distance between shaft 23 and apex 89. At the expanded position shown in the example of Fig. 2, distal-end assembly 40 may have a gap distance 92 between shaft 23 and bump stop 91. In such embodiments, when physician 30 further reduces the distance between ring 54 and apex 89, bump stop 91 limits the expansion of distal-end assembly 40. In other words, bump stop 91 serves as a hard stop, which is configured to prevent the basket shape of distal-end assembly 40 from going completely flat.

In some embodiments, physician 30 may use manipulator 32 (shown in Fig. 1 above) for controlling the distance between apex 89 and ring 54, and therefore the amount of expansion of distal-end assembly 40.

### PRODUCING CATHETER HAVING ABLATION ELECTRODES MADE FROM ELECTRICAL TRACES

Fig. 3 is a flow chart that schematically illustrates a method for producing a basket catheter, in the present example distal-end assembly 40, having ablation electrodes 88 made from electrical traces, in accordance with an embodiment of the present invention.

The method begins at an electrical-trace production step 100, with producing in FPCB 66 one or more electrical traces, at least one of the electrical traces has first and second sections. The first section comprising conductor 77 formed within FPCB 66 and covered by layer 70. The second section that serves as electrode 88, is typically thicker than conductor 77 and has at least surface 84, which is exposed by being formed over outer surface 72 of layer 70 of FPCB 66, as shown and described in detail in Fig. 2 above.

In some embodiments, the first section is made from a gold layer, such as conductor 77 shown in Fig. 2 above, and the second section is made from two or more gold layers, such as layers 82 and 84 shown in inset 60 of Fig. 2 above.

In such embodiments, the layer of conductor 77 (of the first section) and layer 82 (of the second section) are patterned using the same process sequence, whereas layer 80 (and optionally additional layers of the second section), is formed on top of layer 82.

In alternative embodiments, both conductor 77 and electrode 88 are made at the same time by forming an electrical trace (shown in Fig. 2 above) having a thickness 76, and subsequently, thinning (e.g., by etching) conductor 77 to thickness 74. Subsequently, the process comprises producing layer 70 over conductor 77, so as to differ and electrically insulate between conductor 77 and the tissue of heart 26, as described in Fig. 2 above.

Note that in both embodiments, conductor 77 and electrode 88 are made from the same electrical trace (made from gold or any other suitable biocompatible electrically-conductive material) but have different thicknesses 74 and 76, respectively.

At a splines producing step 102, splines 55 are produced by cutting, from the flexible printed circuit board, multiple stripes of FPCB 66. Note that each stripe comprises at least one conductor 77 and at least one electrode 88, as shown and described in detail in Fig. 2 above.

At a distal-end assembly production step 104, splines 55 are assembled together for producing distal-end assembly 40. In some embodiments, the proximal end of each spline 55 is coupled to movable ring 54, and the distal end of each spline 55 is coupled to apex 89.

At a shaft coupling step 106 that concludes the method, distal-end assembly 40 is coupled to shaft 23 for producing catheter 22. In some embodiments, physician 30 may use shaft 23 for expanding and collapsing distal-end assembly 40 by moving ring 54, relative to apex 89, along axis 71, as described in detail in Fig. 2 above.

Although the embodiments described herein mainly address basket catheters, or other sort of expandable catheters, used for cardiac ablation. The methods and systems described herein can also be used in other applications, such as in neurology, otolaryngology, and .renal denervation

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

## Claims

1. A catheter, comprising:
a shaft for insertion into an organ of a patient;
an expandable distal-end assembly, which is coupled to the shaft and comprises multiple splines, wherein at least one of the splines comprises a flexible substrate, which is configured to conform to tissue of the organ; and
at least an electrical interconnection having: (i) a first section, which is formed within the flexible substrate and is configured to conduct ablation signals, and (ii) a second section thicker than the first section, which is formed over an outer surface of the flexible substrate and is configured to apply the ablation signals to the tissue.

2. The catheter according to claim 1, wherein the second section has a thickness larger than 0.1 mm.

3. The catheter according to claim 1, wherein the flexible substrate comprises a flexible printed circuit board (FPCB), and wherein the electrical interconnection comprises an electrical trace made from gold.

4. The catheter according to claim 3, wherein the FPCB comprises at least an electrically insulating layer formed over the first section and configured to electrically insulate between the first section and the tissue.

5. The catheter according to claim 4, wherein the second section has at least a surface, which is not covered by the electrically insulating layer and is configured to apply the ablation signals to the tissue.

6. A method for producing a catheter, the method comprising:
producing, in a flexible substrate, one or more electrical interconnections, at least one of the electrical interconnections is made from an electrically-conductive layer, comprising: (i) a first section formed within the flexible substrate, and (ii) a second section, thicker than the first section, which is formed over an outer surface of the flexible substrate;
cutting one or more stripes of the flexible substrate for producing one or more splines of the catheter; and
assembling the one or more splines to a distal-end assembly of the catheter.

7. The method according to claim 6, wherein assembling the one or more splines comprises coupling (i) a proximal end of the spline to a proximal element of the distal-end assembly, and (ii) a distal end of the spline to a distal element of the distal-end assembly, wherein the proximal element and the distal element are movable relative to one another for expanding and collapsing the distal-end assembly.

8. The method according to claim 6, wherein producing the one or more electrical interconnections comprises producing the second section having a thickness larger than 0.1 mm.

9. The method according to claim 6, wherein producing the electrical interconnection comprises producing electrical traces made from a biocompatible layer.

10. The method according to claim 9, wherein the biocompatible layer comprises gold.

11. The method according to claim 6, wherein the electrically-conductive layer comprises a first sub-layer and a second sub-layer, and wherein producing the electrically-conductive layer comprises: (i) forming the first sub-layer in the first and second sections, and (ii) forming the second sub-layer over the first sub-layer in the second section.

12. The method according to claim 11, wherein the first and second sub-layers are made from gold, and wherein the first and second sub-layers have a combined thickness larger than 0.1 mm.

13. The method according to claim 6, wherein producing the electrically-conductive layer comprises: (i) forming the electrically-conductive layer in the first and second sections, and (ii) thinning the electrically-conductive layer in the first section.

14. The method according to claim 13, wherein the electrically-conductive layer comprises gold and (i) having a thickness larger than 0.1 mm, or (ii) wherein, after the thinning, the first section has a thickness smaller than 0.1 mm.

15. The method according to claim 6, and comprising coupling the distal-end assembly to a shaft of the catheter.

16. The catheter according to claim 1 or the method according to claim 6, wherein the second section is (i) approximately 100 times thicker than the first section or (ii) approximately 20 times thicker than the first section.
